(19) Europäisches Patentamt
European Patent Office
Office européen des brevets

(11) EP 2 479 159 A1

(12) **EUROPEAN PATENT APPLICATION**
published in accordance with Art. 153(4) EPC

(43) Date of publication:
**25.07.2012 Bulletin 2012/30**

(21) Application number: **10817311.3**

(22) Date of filing: **14.09.2010**

(51) Int Cl.:
***C07C 1/24*** *(2006.01)* ***B01J 21/00*** *(2006.01)*
***B01J 23/46*** *(2006.01)* ***C07C 11/113*** *(2006.01)*
***C07C 13/19*** *(2006.01)* ***C07B 61/00*** *(2006.01)*

(86) International application number:
**PCT/JP2010/066275**

(87) International publication number:
**WO 2011/034198 (24.03.2011 Gazette 2011/12)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **17.09.2009 JP 2009215518**
**08.06.2010 JP 2010130751**

(71) Applicant: **Sumitomo Chemical Co., Ltd**
**Chuo-ku, Tokyo 104-8260 (JP)**

(72) Inventors:
- **YOSHII, Masayuki**
**Ichihara-shi**
**Chiba 299-0111 (JP)**
- **NAKAYAMA, Toshio**
**Sodegaura-shi**
**Chiba 299-0245 (JP)**
- **FUKUNAKA, Tadashi**
**Niihama-shi**
**Ehime 792-0009 (JP)**

(74) Representative: **Harmsen, Dirk et al**
**Vossius & Partner**
**Siebertstraße 4**
**DE-81675 München (DE)**

# (54) PREPARATION METHOD FOR COMPOUND PROVIDED WITH DOUBLE BOND

(57) A method for highly selectively producing a compound with a double bond represented by the following formula (III), the method comprising the following steps (A), (B), and (C):
step (A): a step of reacting a compound represented by the following formula (I) and/or a compound represented by the following formula (II) with hydrogen in the presence of 0.1 parts by weight or more of a hydrogenation catalyst, thereby obtaining a reaction liquid,
step (B): a step of removing the hydrogenation catalyst until the amount of the hydrogenation catalyst contained in the reaction liquid obtained in step (A) becomes 0.0010 parts by weight or less, thereby obtaining a hydrogenation catalyst-removed liquid,
step (C): a step of obtaining a compound with a double bond represented by the following formula (III) in the presence of a dehydration catalyst from the hydrogenation catalyst-removed liquid obtained in step (B),

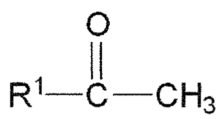
(I)

wherein $R^1$ represents a hydrocarbon group,

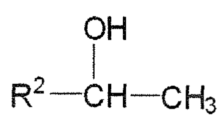
(II)

EP 2 479 159 A1

wherein $R^2$ represents a hydrocarbon group containing an aromatic ring,

$$R^3\text{-CH=CH}_2 \quad (III)$$

wherein $R^3$ represents an alkyl group, or the like.

## Description

### TECHNICAL FIELD

The present invention relates to a method for producing a compound with a double bond by which method a specific compound with a double bond can be obtained highly selectively.

### BACKGROUND ART

Compounds with a double bond are useful as raw materials of olefin polymers. As a method for producing a compound with a double bond, patent documents 1 and 2, for example, each disclose a method of producing a compound with a double bond from a secondary alcohol by using zirconium oxide as a catalyst.
[Patent Document 1] JP 60-158121 A
[Patent Document 2] JP 61-53230 A
However, the methods of producing a compound with a double bond disclosed in patent documents 1 and 2 will result in the generation of two kinds of compounds with a double bond and therefore further improvement with respect to the selectivity of a specific compound with a double bond has been required.

### DISCLOSURE OF THE INVENTION

The problem to be solved by the present invention is to provide a method for producing a specific compound with a double bond by which method a specific compound with a double bond can be obtained highly selectively.
That is, the invention relates to a method for producing a compound with a double bond represented by the following formula (III), the method comprising the following steps (A), (B), and (C):

step (A): a step of reacting a compound represented by the following formula (I) and/or a compound represented by the following formula (II) with hydrogen in the presence of 0.1 parts by weight or more of a hydrogenation catalyst, thereby obtaining a reaction liquid, wherein the whole weight of a feedstock liquid is 100 parts by weight,
step (B): a step of removing the hydrogenation catalyst until the amount of the hydrogenation catalyst contained in the reaction liquid obtained in step (A) becomes 0.0010 parts by weight or less, thereby obtaining a hydrogenation catalyst-removed liquid, wherein the whole weight of the reaction liquid obtained in step (A) is 100 parts by weight,
step (C): a step of obtaining a compound with a double bond represented by the following formula (III) in the presence of a dehydration catalyst from the hydrogenation catalyst-removed liquid obtained in step (B),

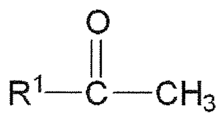

(I)

wherein $R^1$ represents a hydrocarbon group,

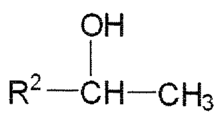

(II)

wherein $R^2$ represents a hydrocarbon group containing an aromatic ring,

$R^3\text{-CH=CH}_2$ (III)

wherein $R^3$ represents an alkyl group, a cycloalkyl group, or an alkyl group substituted by a cycloalkyl group.

### MODE FOR CARRYING OUT THE INVENTION

The method for producing a compound with a double bond represented by the following formula (III) of the present invention involves the following steps (A), (B), and (C):

step (A): a step of reacting a compound represented by the following formula (I) and/or a compound represented

by the following formula (II) with hydrogen in the presence of 0.1 parts by weight or more of a hydrogenation catalyst, thereby obtaining a reaction liquid, wherein the whole weight of a feedstock liquid is 100 parts by weight,
step (B): a step of removing the hydrogenation catalyst until the amount of the hydrogenation catalyst contained in the reaction liquid obtained in step (A) becomes 0.0010 parts by weight or less, thereby obtaining a hydrogenation catalyst-removed liquid, wherein the whole weight of the reaction liquid obtained in step (A) is 100 parts by weight,
step (C): a step of obtaining a compound with a double bond represented by the following formula (III) in the presence of a dehydration catalyst from the hydrogenation catalyst-removed liquid obtained in step (B),

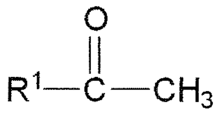
(I)

wherein $R^1$ represents a hydrocarbon group,

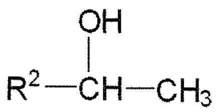
(II)

wherein $R^2$ represents a hydrocarbon group containing an aromatic ring,

$R^3$-CH=$CH_2$ (III)

wherein $R^3$ represents an alkyl group, a cycloalkyl group, or an alkyl group substituted by a cycloalkyl group.

1) Step (A)

Step (A) is a step of reacting a compound represented by the preceding formula (I) and/or a compound represented by the preceding formula (II) with hydrogen in the presence of 0.1 parts by weight or more of a hydrogenation catalyst, thereby obtaining a reaction liquid, wherein the whole weight of a feedstock liquid is 100 parts by weight.
$R^1$ in the compound represented by the above formula (I) represents a hydrocarbon group. Examples of the hydrocarbon group include alkyl groups having from 1 to 20 carbon atoms, aryl groups having from 6 to 20 carbon atoms, and aralkyl groups having from 7 to 20 carbon atoms.
Examples of the alkyl groups having from 1 to 20 carbon atoms include a methyl group, an ethyl group, a n-propyl group, an isopropyl group, a n-butyl group, a sec-butyl group, a tert-butyl group, a n-pentyl group, a neopentyl group, an isopentyl group, a n-hexyl group, a n-heptyl group, a n-octyl group, a n-decyl group, a n-nonyl group, a n-decyl group, a n-dodecyl group, a n-tridecyl group, a n-tetradecyl group, a n-pentadecyl group, a n-hexadecyl group, a n-heptadecyl group, a n-octadecyl group, a n-nonadecyl group, a n-eicosyl group, a cyclohexyl group, and an adamanthyl group.
Examples of the aryl groups having from 6 to 20 carbon atoms include a phenyl group, a 2-tolyl group, a 3-tolyl group, a 4-tolyl group, a 2,3-xylyl group, a 2,4-xylyl group, a 2,5-xylyl group, a 2,6-xylyl group, a 3,4-xylyl group, a 3,5-xylyl group, a 2,3,4-trimethylphenyl group, a 2,3,5-trimethylphenyl group, a 2,3,6-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a pentamethylphenyl group, a 2-ethylphenyl group, a 2,3-diethylphenyl group, a 2,3,4-triethylphenyl group, a 2-n-propylphenyl group, 2-isopropylphenyl group, a 2-n-butylphenyl group, a 2-sec-butylphenyl group, a 2-tert-butylphenyl group, a 2-n-pentylphenyl group, a 2-neopentyl phenyl group, a 2-n-hexylphenyl group, a 2-n-octylphenyl group, a 2-n-decylphenyl group, a 2-n-dodecylphenyl group, a 2-n-tetradecylphenyl group, a naphthyl group, and an anthracenyl group.
Examples of the aralkyl groups having from 7 to 20 carbon atoms include a benzyl group, a (2-methylphenyl)methyl group, a (3-methylphenyl)methyl group, a (4-methylphenyl)methyl group, a (2,3-dimethylphenyl)methyl group, a (2,4-dimethylphenyl)methyl group, a (2,5-dimethylphenyl)methyl group, a (2,6-dimethylphenyl)methyl group, a (3,4-dimethylphenyl)methyl group, a (4,6-dimethylphenyl)methyl group, a (2,3,4-trimethylphenyl)methyl group, a (2,3,5-trimethyl phenyl)methyl group, a (2,3,6-trimethylphenyl)methyl group, a (3,4,5-trimethyl phenyl)methyl group, a (2,4,6-trimethylphenyl)methyl group, a (2,3,4,5-tetramethylphenyl)methyl group, a (2,3,4,6-tetramethylphenyl)methyl group, a (2,3,5,6-tetramethylphenyl)methyl group, a (pentamethylphenyl)methyl group, a (2-ethylphenyl)methyl group, a (2-n-propylphenyl)methyl group, a (2-isopropylphenyl)methyl group, a (2-n-butylphenyl)methyl group, a (2-sec-butylphenyl)methyl group, a (2-tert-butylphenyl)methyl group, a (2-n-pentylphenyl)methyl group, a (2-neopentyl phenyl)methyl group,

a (2-n-hexylphenyl)methyl group, a (2-n-octylphenyl)methyl group, a (2-n-decylphenyl)methyl group, a (2-n-decylphenyl) methyl group, a (2-n-tetradecylphenyl)methyl group, a naphthylmethyl group, an anthracenylmethyl group, a 2-phenylethyl group, a 3-phenylpropyl group, a 4-phenylbutyl group, a diphenylmethyl group, a 2,2-diphenylethyl group, a 3,3-diphenylpropyl group, and a 4,4-diphenylbutyl group.

$R^1$ is preferably a hydrocarbon groups having 2 or more carbon atoms and more preferably a hydrocarbon group having from 2 to 20 carbon atoms.

Examples of the compound represented by the preceding formula (I) include acetone, ethyl methyl ketone, 2-pentanone, 4-methyl-2-pentanone, 2-hexanone, 2-heptanone, acetophenone, and 1-phenyl-2-propanone. From the viewpoint of the usefulness of the compound with a double bond to be obtained, 4-methyl-2-pentanone and acetophenone are preferred. There can be used 4-methyl-2-pentanone prepared by making acetone, obtained as a by-product in a plant for producing resorcinol by aerobically oxidizing m-diisopropylbenzene and then decomposing the resulting peroxide with acid in the presence of an acid catalyst, undergo an aldol reaction.

$R^2$ in the compound represented by the preceding formula (II) represents a hydrocarbon group containing an aromatic ring. Examples of the hydrocarbon group containing an aromatic ring include aryl groups having from 6 to 20 carbon atoms and aralkyl groups having from 7 to 20 carbon atoms. Examples of the aryl groups having from 6 to 20 carbon atoms and the aralkyl groups having from 7 to 20 carbon atoms may be the same as those listed as examples of the aryl groups having from 6 to 20 carbon atoms and the aralkyl groups having from 7 to 20 carbon atoms in the preceding $R^1$.

Examples of the compound represented by the preceding formula (II) include 1-phenylethyl alcohol, 1-benzylethyl alcohol, and 4-phenyl-2-butanol. From the viewpoint of the usefulness of the compound with a double bond to be obtained, 1-phenyl ethyl alcohol is preferred.

As to each of the compound represented by the preceding formula (I) and the compound represented by the preceding formula (II), a single kind of compound may be used alone or alternatively two or more kinds of compounds may be used in combination.

As to the hydrogenation catalyst, any catalyst that can hydrogenate the compound represented by the preceding formula (I) and the compound represented by the preceding formula (II) in the reaction of the compound represented by formula (I) and the compound represented by formula (II) with hydrogen can be used and examples of such a catalyst include nickel, ruthenium, iron, osmium, rhodium, platinum, palladium, copper, and chromium. Among these, metals of Group 8 of the periodic table are preferred from the viewpoint of the reaction yield of hydrogenation. It is also possible to support these metals on a carrier for use as a hydrogenation catalyst. Examples of the carrier include activated carbon, silica, alumina, titania, diatomite, and various types of zeolite.

As the hydrogen, pure hydrogen may be used or alternatively hydrogen containing inert gas such as methane may be used.

The reaction in step (A) may be carried out either with or without use of a solvent. Examples of the solvent include aliphatic hydrocarbons. Examples of such aliphatic hydrocarbons include hexane, cyclohexane, heptane, octane, decane, methylcyclohexane, and ethylcyclohexane.

The reaction temperature is usually from 50 to 200°C and preferably from 70 to 150°C. The reaction pressure is usually from 0.5 to 10 MPa and preferably from 1 to 7 MPa. The reaction time is usually from 0.1 to 24 hours.

Examples of the reaction system in step (A) include a batch system using a tank reactor, a semi-continuous or continuous slurry method, and a continuous fixed bed method using a tubular reactor. As the tank reactor, a single-stage or multistage mixing tank is usually used. Examples of the tubular reactor include fixed bed reactors in which a single unit is arranged or two or more units with a single tube heat exchange structure or a multitubular heat exchange structure having many tubes arranged in parallel are linked in series.

In step (A), the amount to the hydrogenation catalyst used is 0.1 parts by weight or more, where the whole weight of the reaction feedstock liquid is 100 parts by weight. The whole weight of the reaction feedstock liquid as referred to herein means the sum total of the weight of the compound represented by the preceding formula (I) and/or the compound represented by the preceding formula (II) to be used for the reaction in step (A) and the weight of the solvent used for the reaction according to need. If the used amount of the hydrogenation catalyst is less than 0.1 parts by weight, the hydrogenation reaction rate becomes slow, so that it becomes uneconomical. The amount of the hydrogenation catalyst to be used is preferably 0.5 parts by weight or more. The upper limit of the amount of the hydrogenation catalyst to be used, which is not particularly limited, is preferably adjusted to 95 parts by weight or less from an economical point of view.

The used amount of the hydrogenation catalyst is calculated by the following calculation formula in the case of a batch system using a tank reactor:

(charged weight of hydrogenation catalyst)/[(charged weight of compound represented by formula (I) and/or compound represented by formula (II)) + (charged weight of solvent used for reaction according to need)] × 100.

In the case of a semi-continuous or continuous slurry method using a tank reactor, it is calculated by the following formula:

(weight of hydrogenation catalyst in reactor)/(weight of liquid in reactor) × 100

In the case of a continuous fixed bed method using a tubular reactor, it is calculated by the following formula:

(packed amount of hydrogenation catalyst)/{[(feed flow rate of compound represented by formula (I) and/or compound represented by formula (II)) + (feed flow rate of solvent used for reaction according to need)]/(residence time)} × 100.

As a result of the reaction in step (A), a secondary alcohol is obtained.

2) Step (B)

Step (B) is a step of removing the hydrogenation catalyst until the amount of the hydrogenation catalyst contained in the reaction liquid obtained in step (A) becomes 0.0010 parts by weight or less, thereby obtaining a hydrogenation catalyst-removed liquid, wherein the whole weight of the reaction liquid obtained in step (A) is 100 parts by weight. If the hydrogenation catalyst remains in an amount more than 0.0010 parts by weight, by-products will be produced in an increased amount, so that it becomes impossible to produce a specific compound with a double bond economically with a high yield in step (C), which is described below. Preferred is a step of removing the hydrogenation catalyst until the content of the hydrogenation catalyst becomes 0.0004 parts by weight or less, thereby obtaining a hydrogenation catalyst-removed liquid. The method of the removal may be any method by which the hydrogenation catalyst can be removed from the reaction liquid obtained in step (A), examples of which include a washing operation using water or an organic solvent, an adsorption operation, and a filtration operation. Especially, the filtration operation is preferred industrially from the viewpoints of facility and operation because it can be effect by merely passing the reaction liquid through a filter cloth or the like with which the hydrogenation catalyst will be removed. In the case that the reaction system in step (A) is a continuous fixed bed method using a tubular reactor, it sometimes has a function as both step (A) and step (B).

3) Step (C)

Step (C) is a step of obtaining a compound with a double bond represented by the following formula (III) in the presence of a dehydration catalyst from the hydrogenation catalyst-removed liquid obtained in step (B). The hydrogenation catalyst-removed liquid obtained in step (B) contains the secondary alcohol produced by the reaction in step (A), and if the hydrogenation catalyst-removed liquid obtained in step (B) is subjected to this step in the presence of a dehydration catalyst, then the secondary alcohol undergoes dehydration, so that a compound with a double bond represented by the preceding formula (III) is obtained.

$R^3$ in the compound with a double bond represented by the preceding formula (III) represents an alkyl group,

a cycloalkyl group, or an alkyl group substituted with a cycloalkyl group.

Preferred as the alkyl group are alkyl groups having from 1 to 20 carbon atoms. Examples of the alkyl groups having from 1 to 20 carbon atoms include a methyl group, an ethyl group, a n-propyl group, an isopropyl group, a n-butyl group, a sec-butyl group, a tert-butyl group, a n-pentyl group, a neopentyl group, an isopentyl group, a n-hexyl group, a n-heptyl group, a n-octyl group, a n-decyl group, a n-nonyl group, a n-decyl group, a n-dodecyl group, a n-tridecyl group, a n-tetradecyl group, a n-pentadecyl group, a n-hexadecyl group, a n-heptadecyl group, a n-octadecyl group, a n-nonadecyl group, and a n-eicosyl group.

Preferred as the cycloalkyl group are cycloalkyl groups having from 6 to 20 carbon atoms. Examples of the cycloalkyl groups having from 6 to 20 carbon atoms include a cyclohexyl group, a 2-methylcyclohexyl group, a 3-methylcyclohexyl group, a 4-methylcyclohexyl group, a 2,3-dimethylcyclohexyl group, a 2,4-dimethylcyclohexyl group, a2,5-dimethylcyclohexyl group, a 2,6-dimethylcyclohexyl group, a 3,4-dimethylcyclohexyl group, a 3,5-dimethylcyclohexyl group, a 2,3,4-trimethylcyclohexyl group, a 2,3,5-trimethylcyclohexyl group, a 2,3,6-trimethylcyclohexyl group, a 2,4,6-trimethylcyclohexyl group, a 3,4,5-trimethylcyclohexyl group, a 2,3,4,5-tetramethylcyclohexyl group, a 2,3,4,6-tetramethylcyclohexyl group, a 2,3,5,6-tetramethylcyclohexyl group, a pentamethylcyclohexyl group, a 2-ethylcyclohexyl group, a 2,3-diethylcyclohexyl group, a 2,3,4-triethylcyclohexyl group, a 2-n-propylcyclohexyl group, a 2-isopropylcyclohexyl group, a 2-n-butylcyclohexyl group, a 2-sec-butylcyclohexyl group, a 2-tert-butylcyclohexyl group, a 2-n-pentylcyclohexyl group, an adamanthyl group, a 2-neopentylphenyl group, a 2-n-hexylphenyl group, a 2-n-octylphenyl group, a 2-n-decylcyclohexyl group, a 2-n-dodecylcyclohexyl group, a 2-n-tetradecylcyclohexyl group, a decahydronaphthyl group, and a tetradecahydroanthracenyl group.

The alkyl group substituted with a cycloalkyl group is preferably an alkyl group substituted with a cycloalkyl group having from 7 to 20 carbon atoms. Examples of the alkyl group substituted with a cycloalkyl group having from 7 to 20 carbon atoms include a cyclohexylmethyl group, a methyl group (2-methylcyclohexyl), a (3-methylcyclohexyl) methyl group, a (4-methylcyclohexyl)methyl group, a (2,3-dimethylcyclohexyl)methyl group, a (2,4-dimethylcyclohexyl) methyl group, a (2,5-dimethylcyclohexyl)methyl group, a (2,6-dimethylcyclohexyl)methyl group, a (3,4-dimethylcyclohexyl)methyl group, a (4,6-dimethylcyclohexyl)methyl group, a (2,3,4-trimethylcyclohexyl)methyl group, a (2,3,5-trimethyl cyclohexyl)methyl group, a (2,3,6-trimethylcyclohexyl)methyl group, a (3,4,5-trimethylcyclohexyl)methyl group, a (2,4,6-trimethylcyclohexyl)methyl group, a (2,3,4,5-tetramethylcyclohexyl)methyl group, a (2,3,4,6-tetramethylcyclohexyl)methyl group, a (2,3,5,6-tetramethylcyclohexyl)methyl group, a (pentamethylcyclohexyl)methyl group, a (2-ethylcyclohexyl)methyl group, a (2-n-propylcyclohexyl)methyl group, a (2-isopropylcyclohexyl)methyl group, a (2-n-butylcyclohexyl)methyl group, a (2-sec-butylcyclohexyl)methyl group, a (2-tert-butylcyclohexyl)methyl group, a (2-n-pentylcyclohexyl)methyl group, a (2-neopentylcyclohexyl)methyl group, a (2-n-hexylcyclohexyl)methyl group, a (2-n-octylcyclohexyl)methyl group, a (2-n-decylcyclohexyl)methyl group, a (2-n-decylcyclohexyl)methyl group, a (2-n-tetradecylcyclohexyl)methyl group, a decahydronaphthylmethyl group, a tetradecahydroanthracenylmethyl group, a 2-cyclohexylethyl group, a 3-cyclohexylpropyl group, a 4-cyclohexylbutyl group, a dicyclohexylmethyl group, a 2,2-dicyclohexylethyl group, a 3,3-dicyclohexylpropyl group, and a 4,4-dicyclohexylbutyl group.

Examples of the compound with a double bond represented by the preceding formula (III) include propylene, 1-butene, 1-pentene, 4-methyl-1-pentene, 1-hexene, 1-heptene, vinylcyclohexane, allylcyclohexane, and 4-cyclohexyl-1-butene.

The dehydration catalyst may be any catalyst which can cause the secondary alcohol contained in the hydrogenation catalyst-removed liquid obtained in step (B) to undergo dehydration to produce a compound with a double bond represented by the preceding formula (III), examples of which include alumina, silica, silica-alumina, titania, zirconium oxide, and an ion exchange resin. Among these, zirconium oxide is preferred from the viewpoint that a specific compound with a double bond can be obtained in a high selectivity.

The reaction system in step (C) may be either of a liquid phase reaction and a gas phase reaction and examples thereof include a batch system using a tank reactor, a semi-continuous or continuous slurry method, and a continuous fixed bed method using a tubular reactor. As the tank reactor, a single-stage or multistage mixing tank is usually used. Examples of the tubular reactor include fixed bed reactors in which a single unit is arranged or two or more units with a single tube heat exchange structure or a multitubular heat exchange structure having many tubes arranged in parallel are linked in series. In a large-scale industrial operation, it is preferred to carry out the reaction by a fixed bed method from the viewpoints of operativity and economical efficiency.

The reaction in step (C) may be performed using a solvent. Examples of the solvent include aliphatic hydrocarbons and aromatic hydrocarbons. Examples of such aliphatic hydrocarbons include hexane, cyclohexane, heptane, octane, decane, methylcyclohexane, and ethylcyclohexane. Examples of such aromatic hydrocarbons include benzene, toluene, xylene, ethylbenzene, and cumene. Such solvents may be used alone or alternatively may be used as a mixture of two or more of them. The reaction temperature is usually from 100 to 500°C and preferably from 300 to 500°C. The reaction pressure is usually from ordinary pressure to 1 MPa. The reaction time is usually from 0.1 to 10 hours.

The compounds with a double bond represented by the preceding formula (III) to be obtained by the present invention are compounds useful as a raw material of a polymer, a comonomer for polyolefin, and the like, and especially

4-methyl-1-pentene and vinylcyclohexane are compounds useful as a raw material of a heat-resistant polymer, a comonomer for polyolefin, and the like.

EXAMPLES

The present invention will be described below in more detail with reference to examples. The conversions of 4-methyl-2-pentanol and 1-cyclohexylethanol, the selectivities of 4-methyl-1-pentene and vinylcyclohexane, and the selectivities of 4-methyl-2-pentene and ethylidenecyclohexane, which are by-products, are calculated from the amount of a reaction feed, the gas chromatographic compositional analysis thereof, the flow amount of a post-reaction liquid, and the gas chromatographic compositional analysis thereof.

Example 1

(1) Step (A)

To a batch reactor made of metal, a liquid containing 4-methyl-2-pentanone (formula (I)) and ruthenium/alumina (a hydrogenation catalyst containing 5 parts by weight of ruthenium and 95 parts by weight of alumina) are charged so that the content of ruthenium/alumina may become 1.5 parts by weight relative to 100 parts by weight of the liquid, and then a reaction is performed for 10 hours under the condition specified by a liquid temperature of 150°C and a pressure in the reactor of 3 MPa-G with pure hydrogen gas being supplied. The resulting reaction liquid has a 4-methyl-2-pentanol concentration of 99% by weight.

(2) Step (B)

The reaction liquid obtained in the above-described step (1) is left at rest and then a supernatant liquid is filtered through a filter cloth (Zeta Plus 30C, manufactured by Sumitomo 3M Ltd.), so that a hydrogenation catalyst-removed liquid is obtained. The hydrogenation catalyst concentration of the resulting hydrogenation catalyst-removed liquid is less than 0.0001 parts by weight.

(3) Step (C)

To a tubular reactor made of metal (having a sectional area of 0.71 square centimeter) was packed 66 grams of a zirconium oxide catalyst (packed length: 67 cm), and then the hydrogenation catalyst-removed liquid obtained in the above (2) was fed at a rate of 40 g/hour and the temperature of the zirconium oxide catalyst layer was adjusted to 336 to 353°C.

The conversion of 4-methyl-2-pentanol at the reactor outlet was 88%, the selectivity of 4-methyl-1-pentene was 82%, and the selectivity of 4-methyl-2-pentene, which is a by-product, was 11%.

Example 2

(1) Step (A)

To a batch reactor made of metal, a liquid containing 1-phenylethyl alcohol (formula (II)) and acetophenone (formula (I)), and ruthenium/alumina (a hydrogenation catalyst containing 5 parts by weight of ruthenium and 95 parts by weight of alumina) were charged so that the content of ruthenium/alumina might become 1.5 parts by weight relative to 100 parts by weight of the liquid, and then a reaction was performed for 7 hours under the condition specified by a liquid temperature of 80°C and a pressure in the reactor of 3 MPa-G with pure hydrogen gas being supplied. Then, the liquid temperature and the pressure in the reactor were changed to 100°C and 2 MPa-G, respectively, and a reaction was carried out for 4 hours, affording a reaction liquid. The 1-cyclohexylethanol concentration of the resulting reaction liquid was 94% by weight.

(2) Step (B)

The reaction liquid obtained in the above-described step (1) was left at rest and then a supernatant liquid was filtered through a filter cloth (Zeta Plus 30C, manufactured by Sumitomo 3M Ltd.), so that a hydrogenation catalyst-removed liquid was obtained. The hydrogenation catalyst concentration of the resulting hydrogenation catalyst-removed liquid was less than 0.0002 parts by weight.

(3) Step (C)

To a tubular reactor made of metal (having a sectional area of 4.1 square centimeter) was packed 41 grams of a zirconium oxide catalyst (packed length: 10 cm), and then the hydrogenation catalyst-removed liquid obtained in the above (2) was fed in 320 g/hour and the temperature of the zirconium oxide catalyst layer was adjusted to 376 to 398°C.

The conversion of 1-cyclohexylethanol at the reactor outlet was 45%, the selectivity of vinylcyclohexane was 84%, and the selectivity of ethylidenecyclohexane, which is a by-product, was 6%.

Comparative Example 1

In Example 1 (2), the reaction liquid to be obtained in Example 1 (1) is left at rest, affording a supernatant liquid. The hydrogenation catalyst concentration of the resulting supernatant liquid is 0.0018 parts by weight. In Example 1 (3), operations were carried out in the same manner as Example 1 except for feeding the hydrogenation catalyst-removed liquid obtained in Example 1 (2) at a rate of 40 g/hour and the temperature of the zirconium oxide catalyst layer was changed from 336 to 353°C to 337 to 354°C; as a result, the conversion of 4-methyl-2-pentanol at the reactor outlet was 86%, the selectivity of 4-methyl-1-pentene was 75%, and the selectivity of 4-methyl-2-pentene, which is a by-product, was 16%.

Comparative Example 2

In Example 2 (2), the reaction liquid obtained in Example 2 (1) was left at rest, affording a supernatant liquid. The hydrogenation catalyst concentration of the resulting supernatant liquid was less than 0.0014 parts by weight. In Example 2 (3), operations were carried out in the same manner as Example 2 except for feeding the supernatant liquid at a rate of 323 g/hour instead of feeding the hydrogenation catalyst-removed liquid obtained in Example 2 (2) at a rate of 320 g/hour and changing the temperature of the zirconium oxide catalyst layer from 376 to 398°C to 376 to 401°C; the conversion of 1-cyclohexylethanol at the reactor outlet was 47%, the selectivity of vinylcyclohexane was 78%, and the selectivity of ethylidenecyclohexane, which is a by-product, was 12%.

INDUSTRIAL APPLICABILITY

According to the present invention, a specific compound with a double bond can be obtained highly selectively.

## Claims

1. A method for producing a compound with a double bond represented by the following formula (III), the method comprising the following steps (A), (B), and (C):

   step (A): a step of reacting a compound represented by the following formula (I) and/or a compound represented by the following formula (II) with hydrogen in the presence of 0.1 parts by weight or more of a hydrogenation catalyst, thereby obtaining a reaction liquid, wherein the whole weight of a feedstock liquid is 100 parts by weight,
   step (B): a step of removing the hydrogenation catalyst until the amount of the hydrogenation catalyst contained in the reaction liquid obtained in step (A) becomes 0.0010 parts by weight or less, thereby obtaining a hydrogenation catalyst-removed liquid, wherein the whole weight of the reaction liquid obtained in step (A) is 100 parts by weight,
   step (C): a step of obtaining a compound with a double bond represented by the following formula (III) in the presence of a dehydration catalyst from the hydrogenation catalyst-removed liquid obtained in step (B),

   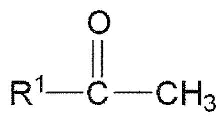

   (I)

   wherein $R^1$ represents a hydrocarbon group,

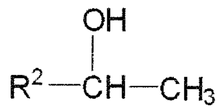

(II)

wherein $R^2$ represents a hydrocarbon group containing an aromatic ring,

$R^3$-CH=$CH_2$ (III)

wherein $R^3$ represents an alkyl group, a cycloalkyl group, or an alkyl group substituted by a cycloalkyl group.

2. The method for producing a compound with a double bond according to claim 1, wherein R1 of formula (I) is a hydrocarbon group having 2 or more carbon atoms.

3. The method for producing a compound with a double bond according to claim 1 or 2, wherein the dehydration catalyst is zirconium oxide.

**INTERNATIONAL SEARCH REPORT**

International application No.
PCT/JP2010/066275

A. CLASSIFICATION OF SUBJECT MATTER
*C07C1/24*(2006.01)i, *B01J21/00*(2006.01)i, *B01J23/46*(2006.01)i, *C07C11/113*(2006.01)i, *C07C13/19*(2006.01)i, *C07B61/00*(2006.01)n

According to International Patent Classification (IPC) or to both national classification and IPC

B. FIELDS SEARCHED

Minimum documentation searched (classification system followed by classification symbols)
C07C1/24, B01J21/00, B01J23/46, C07C11/113, C07C13/19, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2-279643 A (Mitsubishi Petrochemical Co., Ltd.), 15 November 1990 (15.11.1990), claims; examples (Family: none) | 1-3 |
| Y | JP 62-185032 A (Taiho Pharmaceutical Co., Ltd.), 13 August 1987 (13.08.1987), claims; examples (Family: none) | 1-3 |
| Y | JP 62-129231 A (Chisso Corp.), 11 June 1987 (11.06.1987), claims; examples & EP 227250 A1 & DE 3678122 D | 1-3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 November, 2010 (29.11.10) | 07 December, 2010 (07.12.10) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2010/066275

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-103752 A (Arakawa Chemical Industries, Ltd.), 11 April 2000 (11.04.2000), claims 1 to 6 (Family: none) | 1-3 |
| Y | JP 61-130240 A (Sumitomo Chemical Co., Ltd.), 18 June 1986 (18.06.1986), claims 1, 2; page 2, lower column & EP 150832 A2 & DE 3565977 D | 1-3 |
| Y | JP 5-4931 A (Sumitomo Chemical Co., Ltd.), 14 January 1993 (14.01.1993), claims 1, 2; examples (Family: none) | 1-3 |
| A | WO 2007/116906 A1 (Mitsubishi Gas Chemical Co., Inc.), 18 October 2007 (18.10.2007), paragraphs [0024], [0027], [0033]; example 1 & US 2009/0156858 A1 & EP 2003111 A1 | 1-3 |
| A | JP 61-53230 A (Sumitomo Chemical Co., Ltd.), 17 March 1986 (17.03.1986), claims 1, 2 & EP 150832 A2 & DE 3565977 D | 1-3 |
| A | JP 2000-103751 A (Degussa-Hüls AG.), 11 April 2000 (11.04.2000), claims 1 to 6 & US 6441255 B1 & EP 992475 A2 & DE 19844325 A | 1-3 |
| A | JP 2009-120561 A (Sumitomo Chemical Co., Ltd.), 04 June 2009 (04.06.2009), claims 1, 2 (Family: none) | 1-3 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 60158121 A
- JP 61053230 A